# EUROPEAN PATENT SPECIFICATION

(11) **EP 1 257 562 B1**
(45) Date of publication and mention of the grant of the patent: **29.11.2006**
(21) Application number: 01903603.7
(22) Date of filing: 08.02.2001
(51) Int. Cl.: C07K 1/00, B01D 15/08, B01J 20/00

(54) **NOVEL RESIN MATERIALS AND THEIR USE FOR RECOVERING PROTEINS OR PEPTIDES**
NEUE HARZE UND IHRE VERWENDUNG ZUR RÜCKGEWINNUNG VON PROTEINEN ODER PEPTIDEN
NOUVEAUX MATERIAUX RESINIQUES ET LEUR UTILISATION POUR RECUPERER DES PROTEINES OU DES PEPTIDES

(30) Priority: 08.02.2000 DK 200000199; 08.02.2000 US 181079 P
(43) Date of publication of application: 20.11.2002
(73) Proprietor: UPFRONT CHROMATOGRAPHY A/S, DK-2100 Copenhagen Ö (DK)
(72) Inventor: LIHME, Allan, Otto, Fog, DK-3460 Birkerod (DK); HANSEN, Marie, Bendix, DK-2000 Frederiksberg (DK); BISGAARD-FRANTZEN, Hans, DK-2000 Frederiksberg (DK); RAHBEK-NIELSEN, Henrik, DK-3460 Birkerod (DK)
(74) Representative: Plougmann & Vingtoft A/S
(86) International application number: PCT/DK2001/000086
(87) International publication number: WO 2001/058924

(56) References cited:
- WO-A-90/14886
- WO-A-90/15865
- WO-A-92/00799
- WO-A-96/09116
- US-A- 6 090 288
- GUNNAR HOUEN ET AL: "The primary structure and enzymic properties of porcine prochymosin and chymosin" INT. J. BIOCHEM. CELL BIOL., vol. 28, no. 6, 1996, pages 667-675, XP002901712
- BURTON S C ET AL: "One step purification of chymosin by mixed mode chromatography" BIOTECHNOL BIOENG, vol. 56, no. 1, 5 October 1997 (1997-10-05), pages 45-55, XP002901713
- CHEMICAL ABSTRACTS, vol. 131, no. 23, 1999 Columbus, Ohio, US; abstract no. 308212, WONKYUNG K ET AL: "Direct purification of lysozyme from hen egg white using high density mixed mode adsorbent" XP002901714 & J. MICROBIOL. BIOTECHNOL., vol. 9, no. 3, 1999, pages 292-296,
- KENNY SPROULE ET AL: "New stategy for the design of ligands for the purification of pharmaceutical proteins by affinity chromatography" JOURNAL OF CHROMATOGRAPHY B, vol. 740, 2000, XP002901947
- G HOUEN ET AL: "Affinity chromatography of thiol ester-containing proteins" JOURNAL OF CHROMATOGRAPHY A, vol. 799, 1998, pages 139-148, XP002901948
- G H SCHOLZ ET AL: "Salt-independent binding of antibodies form human serum to thiophilic heterocyclic ligands" JOURNAL OF CHROMATOGRAPHY B, vol. 709, 1998, pages 189-196, XP002901949

## Description

### FIELD OF INVENTION

The present invention relates to the field of recovering target proteins or peptides from an aqueous medium by contacting the medium containing the target protein or peptide with a resin that is capable of selectively binding the protein. Specifically, there is disclosed a process of recovering milk clotting enzymes such as chymosin, and other proteins or peptides using novel resin materials.

### TECHNICAL BACKGROUND AND PRIOR ART

Several techniques for recovering target proteins or peptides from aqueous media are available. Such techniques include e.g. ion exchange chromatography, hydrophobic interaction chromatography (HIC) and affinity chromatography. Recently, so-called mixed mode chromatographic resins have become available (see for instance: Burton et al. (1997) Biotechnol. Bioengin. 56, 45-55). This type of resins effects binding of a target molecule under hydrophobic conditions and effects desorption or elution of the target molecule under electrostatic (ionic) or hydrophilic conditions. A recognised problem associated with currently available mixed mode resins is that binding efficiencies of less hydrophobic target molecules to the resin is not very high unless a high salt concentration is used in the aqueous medium containing the target molecule. Another problem with mixed mode resins as it is mentioned in US 5,652,348 is that it may be required to apply a high ligand density (concentration) on the mixed mode chromatographic resin.

Other resin materials are described in the art including the resins as disclosed in WO 96/00735. This application discloses resins useful for the binding of a selected protein or peptide, particularly from an aqueous medium such as a fermentation broth, by hydrophobic interactions between the resin and the selected protein or peptide. The resin is characterised as containing ionisable ligands and/or functionalities which are uncharged at the pH of binding the target protein or peptide. However, in the method as described a high ligand density is claimed as needed for optimal binding and furthermore, selected proteins only bind to the resins when the resin is uncharged. This emphasises that the resin material binds proteins due to hydrophobic interaction resulting in low binding capacities.

Another method is disclosed for the purification of Chymosin (US 5,215,907) by hydrophobic interaction to phenylsepharose resin. Although, the method describes the use of a specific resin for the purification of the protein the result of the method as described is a very low recovery of the protein.

Yet another implication of binding to uncharged resin materials is that the pKa of the resin must be lower than the loading pH of the solvent containing the target protein. The irreversibly inactivation of some proteins e.g. chymosin (Foltmann, B. 1959 Acta. Chem. Scand. 13, 1927-1942.) at neutral pH makes this a serious problem in selection of suitable resins.

In spite of the availability of this multiplicity of general techniques for separating and purifying target molecules such as proteins and peptides from aqueous media, it is generally recognised in the art that when a specific target molecule in a particular aqueous medium is to be recovered at a desired high efficiency, generally available techniques rarely, if ever, provides optimum recovery efficiency for the selected specific target compound.

Accordingly, there is a continuing need to design novel recovery techniques or to adapt available techniques whenever the recovery of a specific target compound in a particular aqueous medium is to be optimised.

The present invention provides such an improved method for recovering target proteins or peptides from aqueous media. The method has proven to be particularly effective in the recovery of chymosin from aqueous media including fermentation media and aqueous extracts containing the chymosin. The invention is based on the discovery that a mixed mode chromatographic resin comprising a solid support matrix having covalently bound thereto a ligand comprising an amine group is highly efficient for recovering chymosin.

In the present invention the ligands have been carefully selected, that have low ligand densities and are capable of binding the target protein with high capacity under conditions where the resin is charged or partially uncharged. Thereby, the above mentioned problems of existing methods has been overcome.

### SUMMARY OF THE INVENTION

Accordingly, in a first aspect there is provided a process for recovering a target protein having milk dotting activity from an aqueous medium containing such a protein, the method comprising the steps of:
(a) contacting said medium with a resin material under conditions permitting the target protein to bind to the resin material, said resin material comprising a solid support matrix and, covalently bound to the matrix, a ligand that is capable of binding the protein, said ligand is benzylamine or a derivative thereof;
and (b) eluting the protein from the resin material.

In a further aspect the invention pertains to a chromatographic resin material comprising a solid support matrix and, covalently bound to the matrix, a ligand that is capable of binding a target protein or peptide, said ligand is benzylamine or a derivative thereof.

In a still further aspect the invention relates to a process of recovering a target protein or peptide from an aqueous medium containing the target protein, the method comprising contacting the aqueous medium with the resin material of the invention under conditions where the target protein or peptide binds to the resin, and changing the conditions such that the target protein or peptide is eluted from the resin.

### DETAILED DISCLOSURE OF THE INVENTION

It is one major objective of the present invention to provide a process for recovering a target protein having milk dotting activity from an aqueous medium containing such a protein.

As used herein the expression "protein having milk dotting activity" refers to any enzyme including proteases that causes milk to clot. Such enzymes are widely used in the manufacture of cheese and are also generally referred to as rennets (primarily milk dotting enzymes of animal origin) and microbial coagulants. The major sources of milk clotting enzymes are animal tissues and micro-organisms naturally producing proteases having milk clotting activity. The major animal rennet enzyme is chymosin (EC 3.4.23.4) an acidic protease that occurs in the stomach where it is excreted as a precursor, prochymosin, that can be activated at pH below 5. Also pepsin (EC 3.4.23.1-3) that is secreted from the gastric mucous membrane as a nonactive proenzyme, pepsinogen, which is activated by the action of the stomach acid, is used as rennet. Additionally, a wide range of micro-organisms, bacteria and filamentous fungi, naturally produce proteases having milk clotting activity. Thus, several *Bacillus* species are sources of such enzymes. Among filamentous fungi, at least the following species are known to produce rennet enzymes: *Rhizomucor pusillus, Rhizomucor miehei* and *Cryphonectria parasitica.* Preparations of such microbial milk clotting enzymes are provided as crude or at least partially purified fermentates of the source organisms. Furthermore, it is contemplated that plant extracts which causes milk clotting is also encompasses by the process of the present invention. Several plant extracts causing milk clotting are described in the art including extraction of the milk clotting enzymes from *Cynara* spp.

In recent years, methods have been developed to produce chymosin recombinantly by inserting a gene coding for the enzyme into suitable microbial host cells and cultivating the host cells under conditions where the gene is expressed, and recovering the enzyme from the fermentation medium (fermentate). A range of host cells for that purpose have been described such as e.g. bacterial strains of *E. coli* and *Bacillus* species, strains of yeast species include *Klyuveromyces lactis, Pichia pastoris, Saccharomyces cerevisiae,* strains of filamentous fungi including e.g. *Aspergillus* species, *Mucor* species or *Rhizomucor* species. The recovery of such recombinantly produced milk clotting enzymes is also encompassed by the present invention.

The process of the invention for recovering from an aqueous medium a target protein as defined above that has milk clotting activity comprises the step of contacting the aqueous medium with a mixed mode chromatographic resin material under conditions permitting the target protein to bind to the resin material. The resin material of the invention comprises a solid support matrix and, covalently bound to the matrix, a ligand as defined hereinbefore that is capable of binding the protein. The term "solid support matrix" refers to the solid backbone material of the resin which contains reactive functionality permitting covalent attachment of the ligand to said backbone material.

The backbone material may be inorganic such as e.g. silica, or organic. Organic backbone materials which are useful herein include as examples cellulose and derivatives hereof, agarose, dextran, polymers such as e.g. polyacrylates, polystyrene, polyacrylamide, polymethacrylate, copolymers. Additionally, ter- and higher polymers can be used provided that at least one of the monomers contains a reactive functionality in the resulting polymer.

Reactive functionalities of the solid support matrix permitting covalent attachment of the ligand group are well known in the art and include e.g. hydroxyl, carboxyl, thiol and amino.

As used herein, the term "ligand" refers to a group consisting of a selected amine as defined herein, and a spacer arm for covalently attaching the ligand to the solid support matrix wherein the amine is preferably capable of being electrostatically charged at one pH and electrostatically uncharged at another pH. The spacer arm can be any group or substituent which is capable of covalently attaching the selected amine to the solid support matrix. Such spacer arms are well known in the art and include e.g. alkylene groups, aromatic groups, alkylaromatic groups, amido groups, amino groups, urea groups, carbamate groups, -R₁-Y-R₂-groups where R₁ and R₂ are alkylene groups and Y is e.g. oxygen or sulfur. One example of a useful spacer arm is epichlorhydrin.

The aqueous medium containing the target protein is contacted with the resin of the invention under conditions permitting the target protein to bind to the resin material. The contact is typically brought about by loading the aqueous medium onto a chromatographic column containing a bed of the resin material. The bed may be a packed bed or an expanded bed or any other bed form which is suitable for the intended purpose. When an expanded bed mode is used, the loading is conveniently made by pumping the medium onto the column either from the bottom or onto the top of the column. The flow rate depends on the volume of the bed and the amount of resin material in the column. A typical flow rate is in the range of 1-20 cm/min such as 5-10 cm/min, e.g. 7.5 cm/min. Such a rate causes the bed of resin material to become fluidised, thus creating an expanded bed.

In useful embodiments, the ligand that binds the target protein is selected so as to obtain that the ligand, under the conditions permitting the target protein to bind to the resin material, can be electrostatically charged or uncharged depending on the buffer pH. Prior to loading the aqueous medium onto the column, the resin material is typically equilibrated to the pH conditions at which the binding of the target protein occurs. The equilibration step is typically carried out by applying a buffer at a pH in the range of 3-10 such as in the range of 5-7.

The pH of the aqueous medium is e.g. pre-adjusted to a pH such as in the range of 3-10 including the range of 5-7. It has been found that the resin materials of the invention are highly effective even at a relatively low density. Thus, it was found that a very high recovery efficiency for chymosin can be achieved by using a resin material that has a concentration (density) of ligand which is at the most 150 µmol per ml of the settled resin material, such as at the most 100 µmol/ml or even at the most 75 µmol/ml.

In preferred embodiments, the process of the invention recovers at least 50% of the total amount of target protein initially present in the aqueous medium, including at least 60%, 70%, 80% or 90% recovery. Even higher recovery rates have been found, such as at least 92.5%, 95%, 97.5% or at least 99%.

The process of the invention for recovering milk clotting enzymes can be used for any of such enzymes as defined above. Thus, the process includes processes wherein the aqueous medium containing the target protein is a microbial fermentation medium, including a medium that has been used for cultivating a recombinant micro-organism capable of expressing a milk clotting enzyme of animal, plant or microbial origin, or an aqueous extract of an animal tissue material. Particularly interesting milk clotting target protein include chymosin, pro-chymosin, pre-pro-chymosin and pepsin. In this context, such animal enzymes can be derived from any animal species naturally producing the enzymes, including a ruminant species such as bovine species, sheep or goats; pigs and camels.

As mentioned above, the enzymes having milk clotting activity can be produced recombinantly e.g. in host cells of fungal species and bacterial species expressing the enzyme. Accordingly, the aqueous medium used in the present process can be a fermentation medium of such recombinant host cells including filamentous species such as *Aspergillus* species, *Rhizomucor* species, *Penicillium* species, and yeast species including *Pichia* species, *Hansenula* species, *Saccharomyces* species and *Kluyveromyces* species. In this context, suitable bacterial host cell species also include *Bacillus* species and *E*. *coli.*

In one preferred embodiment, the solid support matrix is in the form of composite particles comprising a low density component and a high density component. Such particles are disclosed in co-owned WO 92/00799 which is hereby incorporated by reference.

Examples of materials which can be used in such particles as the low density component include hollow glass particles, agarose, cellulose and synthetic polymers such as those mentioned above. As the high density component may be used a material e.g. selected from solid glass particles, ceramic particles and metal particles. The advantages of using such composite solid support matrix materials include that it is possible, by selecting the appropriate ratio between the high density and the low density components, to provide resin materials of the invention having a density that is specifically adapted to the specific requirements defined by the target protein and the aqueous medium containing it. Thus, resin materials that are useful in the present invention may be provided which have a density in the range of 1.01 to 5.0, e.g. in the range of 1.1 to 2.0, 1.2 to 1.8 or 1.3 to 1.7. It is also possible to provide resins having a density below 1.0 such as a density in the range of 0.5 to 0.99 e.g. in the range of 0.6 to 0.9 including the range of 0.7 to 0.8.

The present process of recovering a protein having milk clotting activity is useful for processing aqueous media in large scale volumes under industrial manufacturing conditions. Thus, the process is applicable as a process where the resin material, e.g. in an expanded bed mode, is contained in a column having a total volume of 100 litres or more, such as at least 200 litres, or at least 500 litres, e.g. 1000 litres or even several thousand litres.

In a more specific embodiment, the process of recovering a target protein having milk clotting activity comprises one or more cycles each comprising at least one of the following steps: (i) providing an expandable (fluidisable) bed of the resin material in a chromatographic column at a settled bed height of above 20; (ii) equilibrating the resin material at a pH in the range of 3-10; (iii) loading the aqueous medium onto the column at a linear rate which is in range of 5-10 cm/min to bind the target protein; (iv) washing the loaded column using a washing buffer having a pH in the range of 3-10; (v) eluting the bound target protein from the column by applying onto the column a buffer having a pH below the pl of the target protein, typically a buffer of pH < 7, e.g. < 6, < 5 or < 4, and (vi) regenerating the column under alkaline conditions. Such a process is useful for recovering chymosin and preferably, a selected resin is used that has a chymosin binding capacity of at least 100 International Milk Clotting Units (IMCU)/ml of settled resin, such as at least 1000 IMCU/ml of settled resin, including 5000 IMCU/ml of settled resin, such as 7,500 IMCU/ml of settled resin including at least 10,000 IMCU/ml. A presently preferred ligand for recovering chymosin is benzylamine or a derivative thereof.

It may be preferred to carry out the above process using two chromatographic columns loaded with resin material according to the invention, where the two columns are serially connected. In such an embodiment, the first column is overloaded, i.e. the volume of aqueous medium applied to the column exceeds the maximum binding capacity of the resin material, in order to obtain the highest possible concentration of purified chymosin in the affluent from the first column. The target protein that does not bind to the first column due to the overloading will be bound in the second column. When the first column is fully loaded, the columns are disconnected. The first column is washed, eluted, regenerated and pH adjusted. Then the first column is placed in series after the second column and the second column can now be overloaded with chymosin. Repeated cycles are carried out until substantially all target protein is recovered.

In a further aspect, the invention relates to a selected chromatographic resin material as defined hereinbefore comprising a solid support matrix and, covalently bound to the matrix, a ligand that is capable of binding a target protein or peptide. It is contemplated that such a ligand is useful in a process of recovering a target protein or peptide from an aqueous medium containing the target protein, the method comprising contacting the aqueous medium with the resin material under conditions where the target protein or peptide binds to the resin, and changing the conditions such that the target protein or peptide is eluted from the resin. In one useful embodiment of such a general method, the ligand that is capable of binding a target protein or peptide is benzylamine including derivatives thereof. Target proteins or peptides that can be recovered by such a process include pharmaceutically active proteins or peptides including as examples, peptide hormones, insulin and cytokines, and enzymes not having milk clotting activity such as e.g. other proteases, lipases, phospholipases, carbohydrate degrading enzymes including enzymes that degrade disaccharides and oligosaccharides including starch, cellulose and hemicellulose, which enzymes are typically produced by using recombinant host cells expressing the protein or peptide.

When the processes of the invention is carried out in an expanded bed mode, the column containing the bed may be provided with means for agitating at least part of the fluidised bed or other means for obtaining an even distribution of the particles making up the fluidised bed and thus preventing the occurrence of turbulence or channel formation in the bed. Typically, such agitation means or other means such as a distributor means, e.g. a perforated plate, may be provided at the bottom of the column containing the resin material.

The invention is further illustrated in the following example and the drawings, where
Fig. 1 is a schematic diagram of an Expanded Bed Adsorption (EBA) set-up including the EBA standard conditions used during loading, washing, elution, cleaning-in-place (CIP) and the equilibration step. RV: resin (settled bed) volumes;
Fig. 2. is a graph showing the recovery yield for chymosin and the binding capacity for chymosin of MIMO^{™} 1300 (Upfront Chromatography, Copenhagen, Denmark) (resin with benzylamine as the ligand), respectively vs. the resin bed height at a linear velocity v = 7.5 cm/min;
Fig. 3 shows the recovery for recombinant camel Chymosin;
Fig. 4 Represents a schematic illustration of the EBA set-up for purification of Chymosin and pepsin from calf and ox extracts including conditions used during load, wash, elution, CIP and the equilibration phase;
Fig. 5 illustrates the recovery for animal rennet coagulants using Mimo™1300
Fig. 6 is a graph showing results of a binding study of chymosin to Mimo™1300 at different pH values; and;
Fig. 7 is a graph ilustrating the titration of Mimo^{™}1300 resin with the Benzylamine ligand, Bold line dV/dpH. Fine line ml applied 0.1 M NaOH.

### EXAMPLE 1

### Recovery of chymosin from a fermentation medium

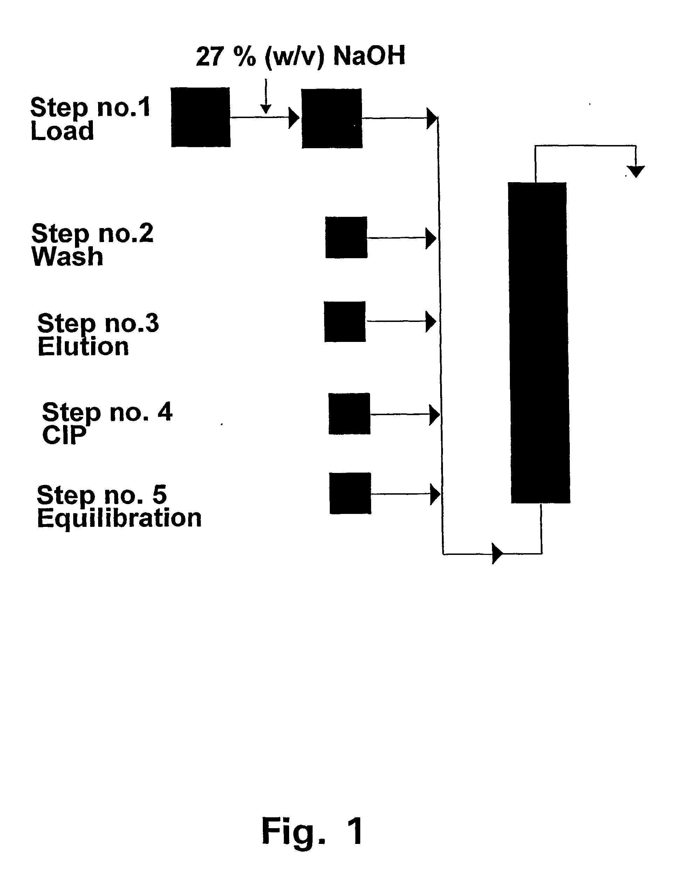

In this experiment, a batch of a fermentate produced by cultivating a recombinant strain of *Aspergillus awamori* expressing bovine chymosin was filtered to substantially remove the fungal biomass and cell debris. The pH of the filtrate was adjusted to about 2. This crude chymosin-containing filtrate was used as the starting material in a recovery process cycle as described in the following (see Fig. 1). Prior to the recovery process, the pH of the acidic filtrate was adjusted to a pH of about 6.0 using 27% (w/w) NaOH.

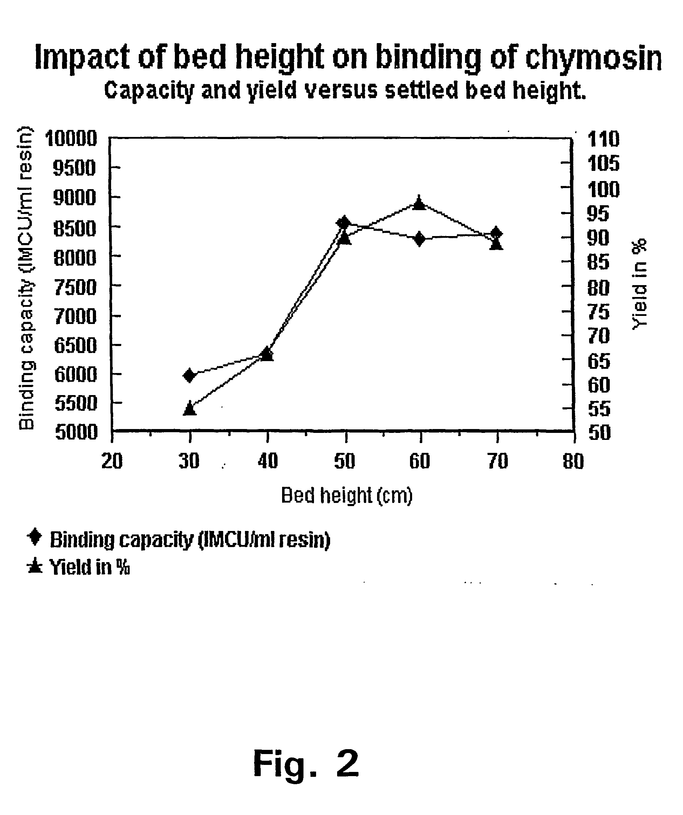

The chymosin was recovered from the thus neutralised filtrate using a chromatographic column containing a bed of MIMO^{™}1300 resin at a settled bed height of about 55 cm. Under the conditions used the resin had a chymosin binding capacity of about 9000 IMCU of chymosin per ml of settled resin. The binding capacity and yield increased with increasing bed high of the resin material as seen in figure 2.

Prior to loading the filtrate onto the column, the resin bed was equilibrated in order to adjust to the pH used during loading. The equilibration was made by applying 25 mM sodium phosphate buffer, pH 5.5.

The loading step (step 1) was carried out by pumping the filtrate into the column at the bottom at a linear flow rate of about 7.5 cm/min and the loading of the column was continued until the binding capacity of the resin bed had been reached or even exceeded, i.e. up to 150% such as 135% of the binding capacity.

Following the loading step, the column was washed to remove any unbound substances from the column using 4 resin (settled) volumes (RV) of 25 mM sodium phosphate buffer, pH 5.5 (step 2).

In a subsequent step, the bound chymosin was eluted (step 3) by applying 4 RV of 150 mM sodium phosphate buffer, pH 2.5 onto the previously washed column.

After the elution step, the resin was subjected to a regeneration (or CIP) step (step 4) to remove remaining substances from the starting material that had not been removed in the elution step. This step was carried out by applying 4 RV of 1 M NaOH (pH > 12) onto the column. In a final step 5, the thus regenerated resin of the regenerated column was subjected to an equilibration step using 4 RV of 250 mM sodium phosphate buffer, pH 5.5.

After this equilibration step the column was ready to be used in a further recovery process cycle such as a cycle as described above.

### EXAMPLE 2

### Purification of recombinant camel chymosin from broth af Aspergillus awamori using expanded bed absorption

In this experiment, The recombinant *Aspergillus awamori* strain expressing the camel coagulant was grown in a complex nutrient medium in a fermentor. After end fermentation the fermentate was filtered to remove the fungal biomass and cell debris. pH of the clear filtrate was subsequently adjusted to pH about 2. This crude filtrate containing the camel Chymosin was used as starting material in the recovery process shown in the figure (Fig. 1). In addition, prior to the recovery process pH of the filtrate was adjusted to approx. pH 6 using 27% (w/w) NaOH.

An expanded bed semi-pilot column (2 cm diameter, 200 cm height) was filled to 70 cm (245 ml) of Mimo^{™}1300 resin. Under the condition used the resin had a dynamic binding capacity of about 5000 IMCU/ml settled resin.

Before loading the filtrate onto the column, the resin was expanded at 7.5 cm/min linear flow rate in equilibration buffer using 25 mM NaPO₄ buffer, pH 6. At the same flow rate filtrate was loaded (Fig. 1, step 1) onto the column and loading was continue until the binding capacity of the resin had been reached or even exceeded.

Following the loading step the resin was then washed (Fig. 1, step 2) with equilibration buffer in order to remove impurities and weak bound substances from the column using 2-3 RV buffer. In the subsequently step (Fig. 1, step 3) camel coagulant was eluted from the resin by applying 4 RV of 122 mM NaPO₄ ; pH 2.5 buffer.

In the following step (Fig. 1, step 4) the resin was subjected to regeneration (or CIP) in order to remove components from the filtrate that had not been removed during the elution step. 5 RV of 1 M NaOH was applied onto the column for the regeneration of the resin. Finally, the resin was subjected to an equilibration step (Fig. 1, step 5) using 3 RV of MQ-water and 6 RV of equilibration buffer. After step 5 the resin was ready to be used in a further recovery cycle as describe above.

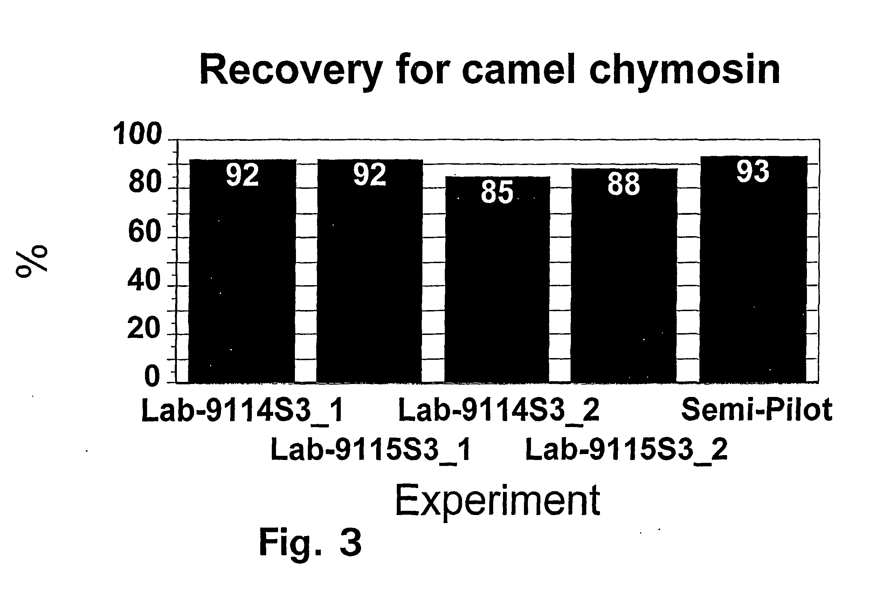

The purification of recombinant camel Chymosin was successfully repeated in 5 purification cycles with recoveries ranging from 85% up to 93% (Fig. 3).

### EXAMPLE 3

### Purification and separation of Chymosin and Pepsin from calf and Ox. stomach (animal rennet)

The following example describes the purification and separation of animal rennet i.e. Chymosin and pepsin from calf and ox. stomach.

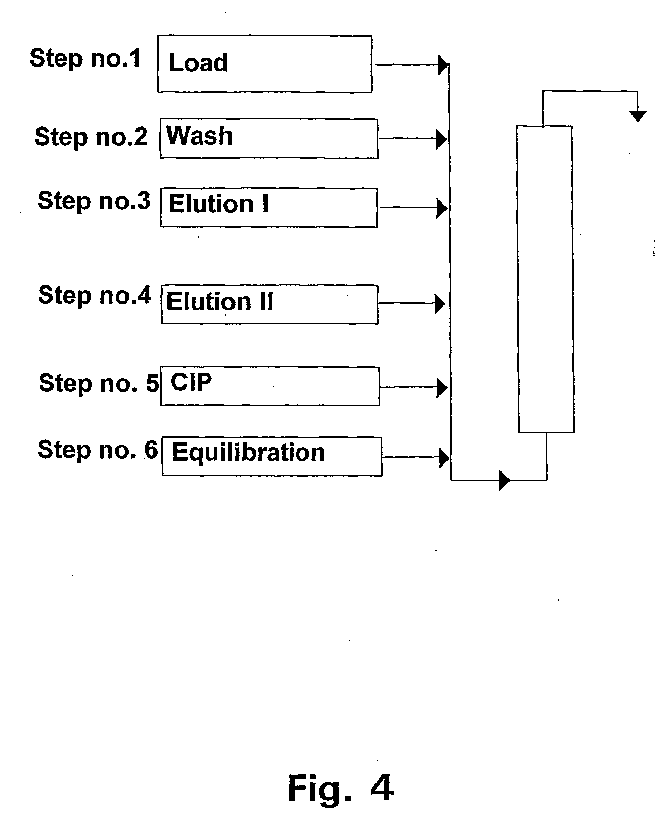

The crude extract used in the process was prepared from a saline extraction at neutral pH of animal stomachs (calf or adult bovine). After end extraction the extract was filtered to remove insoluble particles, biomass and cell debris. The extract containing primary inactive enzymes was subsequently activated adjusting pH to about 2. Coagulants found in the activated animal rennet extract consisted of Chymosin i.e. at least six variants of pl 4.6 - 5.0, mwt 35000, Pepsin, five to seven variants (phosphorylations) of approx. pl 1.6 - 2.0, mwt 35000 and Gastricsin, a minor component, approx. pl 3.5 - 3.8, mwt 35000. Chymosin constituted 20-80%, pepsin of 15-80% and gastricsin 1-5% of the total activity (depending on the type of stomach). Activated extract was used as starting material in the recovery process shown in the figure (Fig. 4).

An expanded bed semi-pilot column (2 cm diameter, 200 cm height) was filled to 50 cm (175 ml) of Mimo^{™} 1300 resin. Before loading the extract onto the column, the resin was expanded at 5.0 cm/min linear flow rate in equilibration buffer using 20 mM H₃PO₄ buffer, pH 5.8. At the same flow rate the rennet extract was loaded (Fig. 4, step 1) onto the column and loading was continue until the binding capacity of the resin had been reached or even exceeded.

Following the loading step the resin was then washed (Fig. 4, step 2) with equilibration buffer in order to remove impurities and weak bound substances from the column using 8-10 RV. In the subsequently step (Fig. 4, Elution I - step 3) primary Chymosin was eluted from the resin by applying 6 -7 RV of 150 mM NaPO₄ ; pH 3.0 buffer, followed by one further elution step (Fig. 4, Elution II - step 4) where primary pepsin was desorbed from the resin using 6 -7 RV of 50 mM HCl.

After elution the resin was subjected to regeneration (Fig. 4, step 5) in order to remove components from the extract that had not been removed during the elution step. 7 RV of 1 M NaOH was applied onto the column for the regeneration of the resin. Finally, the resin was subjected to an equilibration step (Fig. 4, step 6) using 3 RV of MQ-water and 6 RV of equilibration buffer. After step 6 the resin was ready to be used in a further recovery cycle as describe above.

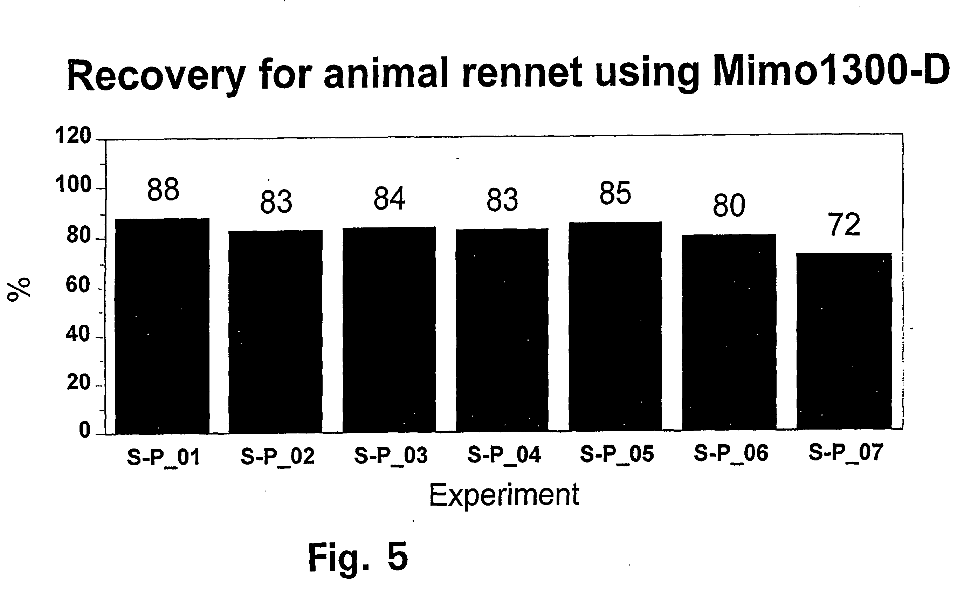

The purification of animal rennet coagulants was successfully repeated in 7 (5 experiments where Chymosin and pepsin were separated and 2 experiments with no separation) purification cycles with recoveries ranging from 72% up to 88% (Fig. 5).

### EXAMPLE 4

### Binding study of Chymosin to Mimo^{™} 1300 at different pH values

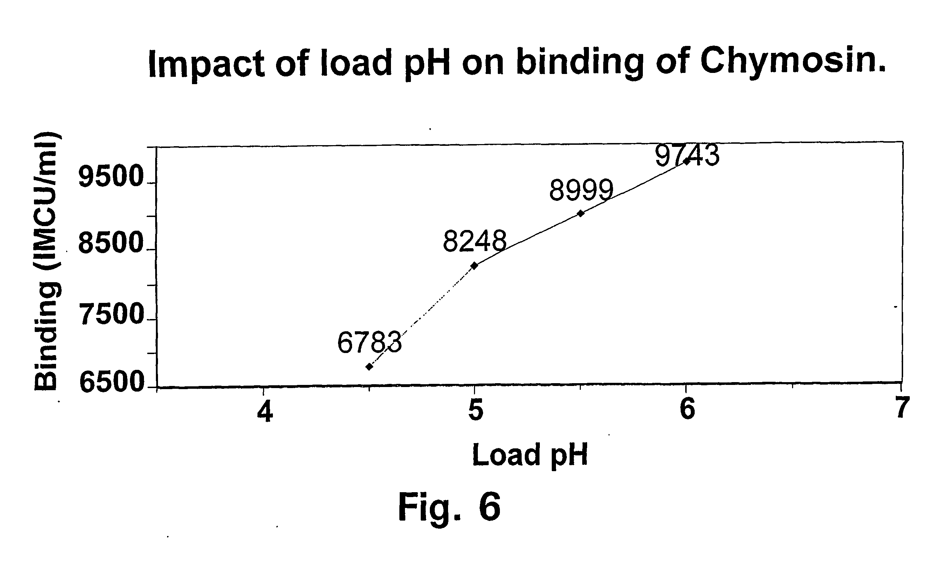

In the present experiment the recombinant *Aspergillus awamori* strain expressing the coagulant was grown in a complex nutrient medium in a fermentor. After end fermentation the fermentate was filtered to remove the fungal biomass and cell debris. pH of the clear filtrate was subsequently adjusted to pH about 2. This crude filtrate containing the coagulant was used as starting material in the binding study of chymosin to Mimo^{™}1300 at different pH values (Fig. 6). Prior to the recovery process pH of the filtrate was adjusted to pH 4.5, 5.0, 5.5 and 6.0, respectively.

An expanded bed semi-pilot column (2 cm diameter, 200 cm height) was filled to 70 cm (245 ml) of Mimo^{™} 1300 resin. Before loading the filtrate onto the column, the resin was expanded at 7.5 cm/min linear flow rate in equilibration buffer using 25 mM NaPO₄ buffer. At the same flow rate, filtrate adjusted to the desired pH value was loaded (Fig. 1, step 1) onto the column. Loading (approximately 10500 IMCU/ml settled resin) was continued until the binding capacity of the resin had been reached or even exceeded.

Following the loading step the resin was then washed (Fig. 1, step 2) with equilibration buffer in order to remove impurities and weak bound substances from the column using 2-3 RV buffer. In the subsequently step (Fig. 1, step 3) chymosin was eluted from the resin by applying 4 RV of 122 mM NaP04 ; pH 2.5 buffer. In the following step (Fig. 1, step 4) the resin was subjected to regeneration (or CIP) in order to remove components from the filtrate that had not been removed during the elution step. 5 RV of 1 M NaOH was applied onto the column for the regeneration of the resin. Finally, the resin was subjected to an equilibration step (Fig. 1, step 5) using 3 RV of MQ-water and 6 RV of equilibration buffer. After step 5 the resin was ready to be used in a further recovery cycle as describe above.

The purification of recombinant chymosin with respect to binding capacity and percentage of chymosin found in the run through fraction at the different load pH values are shown in Figure 8. From Figure 8 it follows that the binding of chymosin onto the resin is possible at all measured pH values. The binding capacity, however, is reduced with decreasing pH, likely a result due to the reduction in the electrostatic interaction between Chymosin (pl 4.5) and the ligand (pKa 7) responsible for the biospecific interaction between Chymosin and the Mimo^{™}1300 resin. Hence, the resin is capable of binding the coagulant under condition where the resin is more than 50% charged during loading.

### EXAMPLE 5

### Feasibility study of Rhizomucor miehei protease purification by Mimo^{™} 1300 resin.

In this example a milk coagulating enzyme was manufactured by submerged fermentation of the fungus *Rhizomucor.* The active milk clotting enzyme is *Rhizomucor miehei* protease (EC 3.4.23.23) which belongs to the aspartyl proteases. The small scale study described in the following was performed to clarify whether the Mimo^{™}1300 resin was feasible for purification of the *Rhizomucor miehei* protease.

A column of 10 ml Mimo^{™} 1300 packed in a 1.5 x 12 cm Econo-Pac column (BioRad, Hercules, CA) was equilibrated with 2 resin volumes (RV) of 80 mM sodium acetate buffer pH 5.5. The solvent flow in the column was approx. 1-2 ml/min, driven by gravity. The Mimo^{™}1300 column was loaded with 18 ml of filtered fermentation medium, preserved with 15% NaCl and 0.4% sodium benzoate (Chr. Hansen A/S, Hørsholm, Denmark). After loading the column was washed with 2 RV of equilibration buffer and eluted with 2 RV of 25 mM Sodium phosphate pH 2.5. Samples were collected during loading, washing and elution of the column. The samples were collected in fractions according to the milk clotting activity determined. The fraction's activity and percentage recovery are presented in the Table below.

**Table 1. Recovery of milk clotting units, in Rhizomucor miehei protease purification by Mimo^{™}1300 resin.**

| Fraction | Volume, ml | Activity, IMCU | % Recovery |
|---|---|---|---|
| Fermentate | 18 | 31.500 | - |
| Run trough | 18 | < 36 | 0 |
| Wash | 20 | 1.900 | 6 % |
| Eluate 1 | 6 | < 12 | |
| Eluate 2 | 10 | 19.859 | 63% |

### Conclusion.

The Mimo^{™}1300 resin is very suitable for purification of *Rhizomucor miehei* protease. An improved recovery, than the obtained 63% of the loaded activity demonstrated in the present experiment, can be achieved when larger resin bed heights are applied (as seen in Fig. 2). Furthermore, since no optimisation of flow, pH and conductivity during loading have been done in this experiment, significant better performance is expected to appear at optimised conditions.

### EXAMPLE 6

### Isolation of chymosin/pepsin from ox stomach extract using chromatography.

Epoxy activated 6 % agarose was coupled with the following amino ligands:
4-(2-aminoethyl)morpholine
1,8-diaminooctan
Octylamine
2-(2-aminoethyl)pyridine
2-aminopyridine
N-phenyl-ethylendiamine
1-phenyl-5-mercapto tetrazol
2-mercaptopyrimidine
Benzylamine
The final ligand concentration of the different matrices was determined by titration with 0.1 N HCl, see results in table 2 below.

**Table 2. Illustrate the ligand concentrations.**

| Ligand | Ligand concentration, µmol/g suction drained matrix |
|---|---|
| 4-(2-aminoethyl)morpholine | 59 |
| 1,8-diaminooctan | 61 |
| Octylamine | 62 |
| 2-(2-aminoethyl)pyridine | 67 |
| 2-aminopyridine | 25 |
| N-phenyl-ethylendiamine | 40 |
| 1-phenyl-5-mercapto tetrazol | 40 |
| 2-mercaptopyrimidine | 20 |
| Benzylamine | 63 |

### Materials and methods.

Extract from ox. stomach containing pepsin and chymosin was activated by adjusting pH to 2.2 with 1 M HCl. After 30 minutes the pH was increased to 5.7 with 1 M NaOH. The extract was clarified by the use of filter help. The activity of the extract was determined to be 25 IMCU/ml extract.

Each matrix was equilibrated with tap water. 0.5 ml of the respective matrices was incubated for 1 hour with 50 ml of the clarified extract (pH 5.7).
After incubation the matrix was collected and transferred to a chromatographic column. The non-bound material was washed out of the matrices with tap water.
The bound proteins were eluted by adding 50 mM HCl. The eluate was neutralised with 1 M NaOH.

### Results.

The capacity (illustrated in table 3) of the different matrices was estimated by milk clotting assay on the supernatants after incubation with the matrices.

**Table 3. Capacity of different ligands.**

| Ligand | Capacity |
|---|---|
| 4-(2-aminoethyl)morpholine | + |
| 1,8-diaminooctan | +++ |
| Octylamine | +++ |
| 2-(2-aminoethyl)pyridine | ++ |
| 2-aminopyridine | 0 |
| N-phenyl-ethylendiamine | +++ |
| 1-phenyl-5-mercapto tetrazol | 0 |
| 2-mercaptopyrimidine | 0 |
| Benzylamine | +++ |

| | |
|---|---|
| +++ bound more than 90 % of the applied enzyme ++ bound 50-90 % of the applied enzyme + bound 10-50 % of the applied enzyme 0 bound less than 10 % of the applied enzyme | |

### Recovery of enzymes.

The amount of eluted enzymes from each matrix was estimated by milk clotting assay. See results in table 4 below.

**Table 4. Recovery of milk clotting enzymes.**

| Ligand | |
|---|---|
| 4-(2-aminoethyl)morpholine | +++ |
| 1,8-diaminooctan | + |
| Octylamine | + |
| 2-(2-aminoethyl)pyridine | +++ |
| 2-aminopyridine | - |
| N-phenyl-ethylendiamine | +++ |
| 1-phenyl-5-mercapto tetrazol | - |
| 2-mercaptopyrimidine | - |
| Benzylamine | +++ |

| | |
|---|---|
| - no results. + 0-25 % of the bound enzymes were recovered in the eluate. +++ >90 % of the bound enzymes were recovered in the eluate. | |

### EXAMPLE 7

### Determination of ligand densities and pKa values of Benzylamine, Morpholinamine and Octylamine resins.

Preparation of resin. The resin was rinsed in a glass filter funnel with 150 ml solution of 0.1 M NaOH, 0.1 M HCl and 1 M NaCl. Between each solution the resin was rinsed with degassed Milli-Q water (MQW). For titration, 5 g of resin was weighted into a 100 ml container and suspended in 30 ml MQW. The resin was titrated with 0.1 M NaOH on a Methrohm 716 DMS Tritrino, using volume increments of 0.020 ml, tolerating 3 mV signal drift and equilibration time of 600 sec. Prior to titration, the 0.1 M NaOH solution was prepared from NaOH pellets and the resin suspension was pH adjusted with 100 µl 0.5 M HCl.

### Results

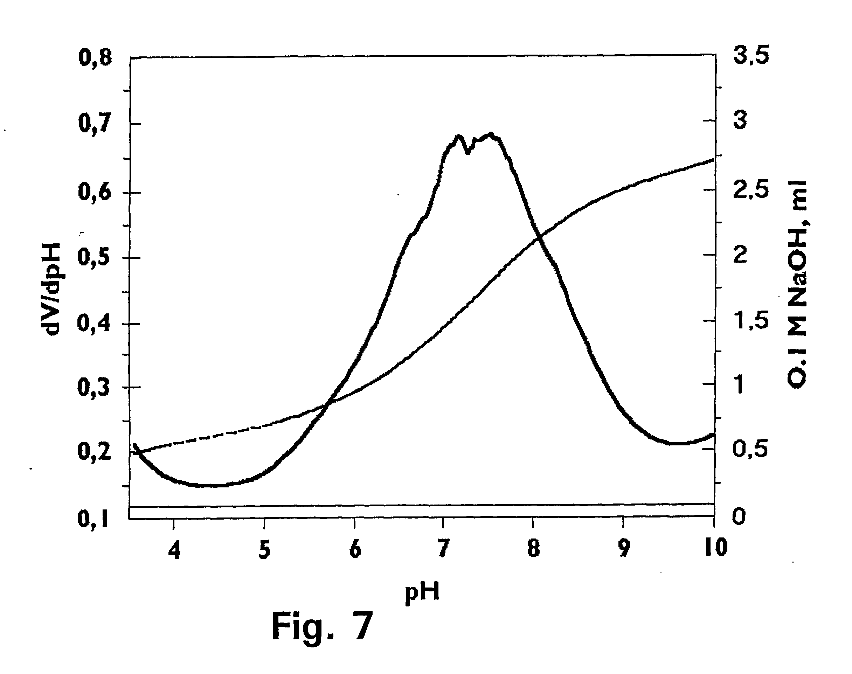

Using the base consumption from the titration of the three resins the ligand densities were calculated and the following ligand densities were obtained: 36 µmol benzylamine/g resin, 96 µmol 4-(2-aminoethyl)morpholine/g resin, and 35 µmol octylamine/g resin. The pKa values were estimated from dV/dpH plotted as function of pH. Estimates of pKa were 7.5, 5.5 and 7.7 for benzylamine resin, 4-(2-aminoethyl)morpholine resin and octylamine resin respectively. From the titration curves presented (Figs. 7, 8 and 9) it is obvious that a significant portion of the different resins will be charged if the resins are operated below pH 7

## Claims

1. A process for recovering a target protein having milk clotting activity from an aqueous medium containing such a protein, the method comprising the steps of
(i) contacting said medium with a resin material under conditions permitting the target protein to bind to the resin material, said resin material comprising a solid support matrix and, covalently bound to the matrix, a ligand that is capable of binding the protein, said ligand is benzylamine, and
(ii) eluting the protein from the resin material.

2. A method according to claim 1 wherein, under the conditions permitting the target protein to bind to the resin material, a proportion of the ligand which is in the range of 5-100% is electrostatically charged.

3. A method according to claim 1 wherein the resin material has a ligand concentration which is at the most 100 µmol per ml of resin material.

4. A method according to claim 1 wherein at least 90% of the total amount of target protein initially present in the aqueous medium is recovered.

5. A process according to claim 1 wherein the aqueous medium containing the target protein is selected from the group consisting of a microbial fermentation medium and an aqueous extract of an animal or plant tissue material.

6. A process according to claim 5 wherein the fermentation medium is a medium used for cultivating a recombinant micro-organism capable of expressing a milk clotting enzyme of animal origin, the cultivation is under conditions where the enzyme is expressed.

7. A process according to claim 6 wherein the milk clotting enzyme Is selected from the group consisting of chymosin, pro-chymosin, pre-pro-chymosin and pepsin.

8. A process according to claim 6 wherein the recombinant micro-organism is selected from the group consisting of a fungal species and a bacterial species.

9. A process according to claim 8 wherein the fungal species is selected from the group consisting of an *Aspergillus* species, a *Rhizomucor* species, a *Penicillium* species and a yeast species including a *Pichla* species, a *Hansenula* species, a *Saccharomyces* species and a *Kluyveromyces* species.

10. A process according to claim 1 wherein the target protein having milk clotting activity is derived from a microbial species including a *Bacillus* species.

11. A process according to claim 1 wherein the resin material is in the form of composite particles comprising a low density component and a high density component.

12. A process according to claim 11 wherein the low density component comprises a material selected from the group consisting of hollow glass particles, agarose, cellulose and a synthetic polymer.

13. A process according to claim 11 or 12 wherein the high density component comprises a material selected from the group consisting of solid glass particles, ceramic particles and metal particles.

14. A process according to claim 11 wherein the density of the composite particles is in the range of 1.01 to 5.0.

15. A process according to claim 11 wherein the density of the composite: particles is In the range of 0.5 to 0.99.

16. A process according to claim 1 wherein the resin material is contained in a column having a total volume of 100 litres or more.

17. A process according to claim 1 wherein, under the conditions permitting the target protein to bind to the resin material, the ligand is essentially electrostatically uncharged and, under the conditions where the target protein is eluted, the ligand is electrostatically charged.

18. A process according to claim 1 wherein, under the conditions permitting the target protein to bind to the resin material, the ligand is essentially electrostatically charged and, under the conditions where the target protein is eluted, the ligand is electrostatically uncharged.

19. A process according to claim 1 comprising one or more cycles comprising the following steps:
(i) providing a bed of the resin material in a chromatographic column at a settled bed height of above 20 cm,
(ii) equilibrating the resin material at a pH in the range of 3-10,
(iii) loading the aqueous medium onto the column at a linear rate which is in range of 3-10 cm/min to bind the target protein;
(iv) washing the loaded column using a washing buffer having a pH in the range of 3-7;
(v) eluting the bound target protein from the column by applying onto the column a buffer having a pH below the pI of the target protein, typically a buffer pH < 4, and
(vi) regenerating the column under alkaline conditions.

20. A process according to claim 1 or 19 wherein the resin material has a chymosin binding capacity of at least 5,000 IMCU/ml.

21. A chromatographic resin material comprising a solid support matrix and, covalently bound to the matrix, a ligand that is capable of binding a target protein or peptide, said ligand is benzylamine.

22. A process of recovering a target protein or peptide from an aqueous medium containing the target protein, the method comprising contacting the aqueous medium with the resin material of claim 21 under conditions where the target protein or peptide binds to the resin, and changing the conditions such that the target protein or peptide is eluted from the resin.

23. A process according to claim 22 wherein the target protein or peptide is a pharmaceutically active protein or peptide.

24. A process according to claim 22 wherein the target protein or peptide is an enzyme not having milk dotting activity.

25. A process according to claim 22 wherein the resin material is an expanded fluidised bed contained in a chromatographic column.

26. A process according to claim 22 wherein resin material contained in the chromatographic column is in a fluidised (expanded) bed state and the column is provided with means for agitating at least part of the fluidised bed.

## Patentansprüche

1. Verfahren zur Wiederherstellung eines Zielproteins mit Milchgerinnungsaktivität aus einem wässrigen Medium, das ein solches Protein enthält, wobei das Verfahren folgende Schritte umfasst:
(i) Kontaktierung des Mediums mit einem Harzmaterial unter Bedingungen, die das Anbinden des Zielproteins an das Harzmaterial erlauben, wobei das Harzmaterial eine feste Trägermatrix und einen an die Matrix kovalent gebundenen Ligand umfasst, der das Protein binden kann, wobei der Ligand Benzylamin ist, und
(ii) Eluierung des Proteins vom Harzmaterial.

2. Verfahren nach Anspruch 1, worin, unter den Bedingungen, die das Anbinden des Zielproteins an das Harzmaterial erlauben, ein Teil des Ligands, der innerhalb des Bereichs von 5-100% liegt, elektrostatisch geladen ist.

3. Verfahren nach Anspruch 1, worin das Harzmaterial eine Ligandkonzentration hat, die höchstens 100 µmol pro ml Harzmaterial ist.

4. Verfahren nach Anspruch 1, worin mindestens 90% der Gesamtmenge des Zielproteins, die ursprünglich im wässrigen Medium vorhanden war, wiederhergestellt ist.

5. Verfahren nach Anspruch 1, worin das wässrige Medium, das das Zielprotein enthält, aus der Gruppe bestehend aus einem mikrobiellen Fermentierungsmedium und einem wässrigen Extrakt eines Tier- oder Pflanzengewebematerials ausgewählt ist.

6. Verfahren nach Anspruch 5, worin das Fermentierungsmedium ein Medium ist, das zur Kultivierung eines rekombinanten Mikroorganismus verwendet wird, das imstande ist, ein Milchgerinnungsenzym tierischen Ursprungs auszudrücken, indem die Kultivierung unter Bedingungen erfolgt, wo das Enzym ausgedrückt ist.

7. Verfahren nach Anspruch 6, worin das Milchgerinnungsenzym aus der Gruppe bestehend aus Chymosin, pro-Chymosin, pre-pro-Chymosin und Pepsin ausgewählt ist.

8. Verfahren nach Anspruch 6, worin der rekombinante Mikroorganismus aus der Gruppe bestehend aus einer Pilzspecies und einer Bakterienspecies ausgewählt ist.

9. Verfahren nach Anspruch 8, worin die Pilzspecies aus der Gruppe bestehend aus einer *Aspergillus*-Species, einer *Rhizomucor*-Species, einer *Penicillium*-Species und einer Hefespecies, hierunter einer Pichia-Species, einer *Hansenula*-Species, einer *Saccharomyces*-Species und einer *Kluyveromyces*-Species, ausgewählt ist.

10. Verfahren nach Anspruch 1, worin das Zielprotein mit Milchgerinnungsaktivität aus einer mikrobiellen Species, hierunter einer *Bacillus*-species, abgeleitet ist.

11. Verfahren nach Anspruch 1, worin das Harzmaterial in Form von Verbundpartikeln ist, die eine Komponente mit niedriger Dichte und eine Komponente mit hoher Dichte umfassen.

12. Verfahren nach Anspruch 11, worin die Komponente mit niedriger Dichte ein Material umfasst, das aus der Gruppe bestehend aus hohlen Glaspartikeln, Agarose, Zellulose und einem synthetischen Polymer ausgewählt ist.

13. Verfahren nach Anspruch 11 oder 12, worin die Komponente mit hoher Dichte ein Material umfasst, das aus der Gruppe bestehend aus festen Glaspartikeln, keramischen Partikeln und Metallpartiklen ausgewählt ist.

14. Verfahren nach Anspruch 11, worin die Dichte der Verbundpartikel innerhalb des Bereichs von 1,01 bis 5,0 liegt.

15. Verfahren nach Anspruch 11, worin die Dichte der Verbundpartikel innerhalb des Bereichs von 0,5 bis 0,99 liegt.

16. Verfahren nach Anspruch 1, worin das Harzmaterial in einer Säule mit einem Gesamtvolumen von 100 Litern oder mehr enthalten ist.

17. Verfahren nach Anspruch 1, worin, unter den Bedingungen, die das Anbinden des Zielproteins an das Harzmaterial erlauben, der Ligand im wesentlichen elektrostatisch ungeladen ist, und, unter den Bedingungen, worin das Zielprotein eluiert wird, der Ligand elektrostatisch geladen ist.

18. Verfahren nach Anspruch 1, worin, unter den Bedingungen, die das Anbinden des Zielproteins an das Harzmaterial erlauben, der Ligand im wesentlichen elektrostatisch geladen ist, und, unter den Bedingungen, worin das Zielprotein eluiert wird, der Ligand elektrostatisch ungeladen ist.

19. Verfahren nach Anspruch 1, umfassend ein oder mehrere Zyklen, umfassend folgende Schritte:
(i) Bereitstellung eines Bettes aus Harzmaterial in einer chromatografischen Säule in einer eingestellten Betthöhe von mehr als 20 cm,
(ii) Äquilibrierung des Harzmaterials bei einem pH-Wert im Bereich von 3-10,
(iii) Beladen des wässrigen Mediums auf die Säule mit einer lineären Geschwindigkeit, die im Bereich von 3-10 cm/Min, liegt, um das Zielprotein zu binden;
(iv) Waschen der beladenen Säule unter Anwendung eines Waschpuffers mit einem pH-Wert im Bereich von 3-7;
(v) Eluierung des gebundenen Zielproteins von der Säule, indem auf der Säule ein Puffer mit einem pH-Wert unter dem pI des Zielproteins, typisch ein Puffer mit pH < 4, angebracht wird, und
(vi) Regenerierung der Säule unter alkalischen Bedingungen.

20. Verfahren nach Anspruch 1 oder 19, worin das Harzmaterial eine chymosinbindende Kapazität von mindestens 5.000 IMCU/ml hat.

21. Chromatografisches Harzmaterial, das eine feste Trägermatrix und einen an die Matrix kovalent gebundenen Ligand umfasst, der imstande ist, an das Zielprotein oder -peptid zu binden, indem der Ligand Benzylamin ist.

22. Verfahren zur Wiederherstellung eines Zielproteins oder -peptids aus einem wässrigen Medium, das das Zielprotein enthält, wobei das Verfahren das Kontaktieren des wässrigen Mediums mit dem Harzmaterial nach Anspruch 21 umfasst, unter Bedingungen, wo das Zielprotein oder -peptid an das Harz bindet, und indem die Bedingungen so verändert werden, dass das Zielprotein oder -peptid vom Harz eluiert wird.

23. Verfahren nach Anspruch 22, worin das Zielprotein oder -peptid ein pharmazeutisch aktives Protein oder Peptid ist.

24. Verfahren nach Anspruch 22, worin das Zielprotein oder -peptid ein Enzym ohne Milchgerinnungsaktivität ist.

25. Verfahren nach Anspruch 22, worin das Harzmaterial ein expanded fluidisiertes Bett ist, das in einer chromatographischen Säule enthalten ist.

26. Verfahren nach Anspruch 22, worin das Harzmaterial, das in der chromatographischen Säule enthalten ist, sich in einem fluidisierten (expanded) Bettzustand befindet, worin die Säule mit Mitteln bereitgestellt wird, um zumindest einen Teil des fluidisierten Bettes umzurühren.

## Revendications

1. Procédé de récupération d'une protéine cible ayant une activité de coagulation du lait à partir d'un milieu aqueux contenant une telle protéine, le procédé comprenant les étapes de
(i) mise en contact du milieu avec un matériau de résine dans des conditions permettant à la protéine cible de se fixer au matériau de résine, le matériau de résine comprenant une matrice de support solide et, un ligand fixé de manière covalente à la matrice et capable de fixer la protéine, le ligand étant l'amine benzylique, et
(ii) élution de la protéine du matériau de résine.

2. Procédé selon la revendication 1, dans lequel une proportion du ligand dans une plage de 5 à 100%, dans des conditions permettant à la protéine de se fixer au matériau de résine, est chargé électrostatiquement .

3. Procédé selon la revendication 1, dans lequel le matériau de résine a une concentration en ligand qui est au plus 100 µmol par mi de matériau de résine.

4. Procédé selon la revendication 1, dans lequel au moins 90% du taux total de protéine cible initialement présent dans le milieu aqueux est récupéré.

5. Procédé selon la revendication 1, dans lequel le milieu aqueux contenant la protéine cible est choisi dans le groupe constitué d'un milieu de fermentation microbien et un extrait aqueux de tissu d'animal ou de plante.

6. Procédé selon la revendication 5, dans lequel le milieu de fermentation est un milieu utilisé pour cultiver un microorganisme recombinant capable d'exprimer une enzyme de coagulation du lait d'origine animale, la culture étant sous des conditions dans lesquelles l'enzyme est exprimée.

7. Procédé selon la revendication 6, dans lequel l'enzyme de coagulation du lait est choisie dans le groupe constitué de chymosine, pro-chymosine, pre-pro-chyrnosine et de pepsine.

8. Procédé selon la revendication 6, dans lequel le microorganisme recombinant est choisi dans le groupe constitué d'espèces fongiques et d'espèces bactériens.

9. Procédé selon la revendication 8, dans lequel les espèces fongiques sont choisies dans le groupe constitué d'une espèce *Aspergillus,* une espèce *Rhizomucor,* une espèce *Penicillium* et une espèce de levure comprenant une espèce *Pichia,* une espèce *Hansenula,* une espèce *Saccharomyces* et une espèce *Kluyveromyces.*

10. Procédé selon la revendication 1, dans lequel la protéine cible ayant l'activité de coagulation du lait est dérivée d'une espèce microbienne comprenant une espèce *Bacillus.*

11. Procédé selon la revendication 1, dans lequel le matériau de résine est sous la forme de particules composites comprenant un élément de basse densité et un élément de haute densité.

12. Procédé selon la revendication 11, dans lequel l'élément de basse densité comprend un matériau choisi dans le groupe constitué de particules de verre creuses, l'agarose, la cellulose et un polymère synthétique.

13. Procédé selon la revendication 11 ou 12, dans lequel l'élément de haute densité comprend un matériau choisi dans le groupe constitué de particules de verre solides, des particules céramiques et des particules métalliques.

14. Procédé selon la revendication 11, dans lequel la densité des particules composites est dans la plage de 1,01 à 5,0.

15. Procédé selon la revendication 11, dans lequel la densité des particules composites est dans la plage de 0,5 à 0,99.

16. Procédé selon la revendication 1, dans lequel le matériau de résine est contenu dans une colonne ayant un volume totale de 100 litres ou plus.

17. Procédé selon la revendication 1, dans lequel le ligand, sous les conditions permettant à la protéine cible de se fixer au matériau de résine, est essentiellement non chargé électrostatiquement et ou le ligand, sous les conditions sous lesquelles la protéine cible est éluée, est chargé électrostatiquement.

18. Procédé selon la revendication 1, dans lequel le ligand, sous les conditions permettant à la protéine cible de se fixer au matériau de résine, est essentiellement chargé électrostatiquement et ou le ligand, sous les conditions sous lesquelles la protéine cible est éluée, est non chargé électrostatiquement.

19. Procédé selon la revendication 1, comprenant un ou plusieurs cycles comprenant les étapes suivantes:
(i) mise au point d'un lit du matériau de résine dans une colonne chromatographique à une hauteur de lit stationnaire au-dessus de 20 cm,
(ii) mise en équilibre du matériau de résine à un pH dans la plage de 3 à 10,
(iii) chargement du milieu aqueux sur la colonne à une vitesse linéaire dans la plage de 3 à 10 cm/min pour fixer la protéine cible ;
(iv) lavage de la colonne chargée en utilisant un tampon de lavage dont le pH est dans la plage de 3 à 7;
(v) élution de la protéine cible fixée de la colonne en appliquant à la colonne un tampon ayant un pH au-dessous du pI de la protéine cible, généralement un tampon pH < 4, et
(vi) régénération de la colonne sous des conditions alcalines.

20. Procédé selon la revendication 1 ou 19, dans lequel le matériau de résine a une capacité de fixation de chymosine d'au moins 5,000 IMCU/ml.

21. Matériau de résine chromatographique comprenant une matrice de support solide et, un ligand fixé de manière covalente à la matrice et capable de fixer la protéine cible ou le peptide cible, le ligand étant l'amine benzylique.

22. Procédé de récupération d'une protéine cible ou un peptide cible d'un milieu aqueux contenant la protéine cible, le procédé comprenant la mise en contact du milieu aqueux avec le matériau de résine selon la revendication 21 dans des conditions sous lesquelles la protéine cible ou le peptide cible se fixent à la résine, et les changements des conditions tels que la protéine cible ou le peptide cible sont élués de la résine.

23. Procédé selon la revendication 22, dans lequel la protéine cible ou le peptide cible est une protéine ou un peptide pharmaceutiquement active ou actif.

24. Procédé selon la revendication 22, dans lequel la protéine cible ou le peptide cible est une enzyme sans activité de coagulation du lait.

25. Procédé selon la revendication 22, dans lequel le matériau de résine est un lit fluidisé détendu contenu dans une colonne chromatographique.

26. Procédé selon la revendication 22, dans lequel le matériau de résine contenu dans la colonne chromatographique est dans un état de lit fluidisé (détendu), et la colonne est munie de moyens d'agitation d'au moins une partie du lit fluidisé.
